# EUROPEAN PATENT APPLICATION

(11) **EP 1 974 680 A1**
(43) Date of publication of application: **01.10.2008**
(21) Application number: 08380080.5
(22) Date of filing: 12.03.2008
(51) Int. Cl.: A61B 17/16, A61C 8/00

(54) **Removable coupling system for motor operated surgical tools**

(30) Priority: 26.03.2007 ES 200700786
(71) Applicant: Soadco, S.L., Escaldes-Engordany (AD)
(72) Inventor: Padros Fradera, Alejandro, 08006 Barcelona (ES)
(74) Representative: SUGRANES - VERDONCES - FERREGÜELA

(57) **Abstract**

A removable coupling system (10) for motor operated surgical tools. A removable coupling system for motorised surgical tools is presented, these being of the type comprised of two complementary coupling items: a motor shaft (20) and a tool head (2). The motor shaft and the tool head are linked together by an intermediate coupling rod (1), this being coupled at one end to the motor shaft, and receiving the coupling of the tool head at the other, the intermediate rod and the tool head being provided, at their coupling ends, with corresponding mutual fitting items, constituted of a male (3) and a female (4) section respectively, which when fitted together are adjustedly coupled together, with no possibility of rotation between the two, and in that it comprises an elastic means (6) complementary to a retaining means (7) integrated respectively in the intermediate rod and in the tool head, or vice versa, in such a way that during the coupling operation the elastic means is press-fitted into the retaining means until it reaches a final coupled position in which the retaining means presents resistance to the axial separation of the two sections.

## Description

### Technical field of the invention

This invention relates to a removable coupling system for the execution of milling, threading, screwing on and/or unscrewing operations, for motor operated surgical tools.

### Background of the invention

In current orthodontic treatments, and more specifically in the mouth surgery, the technique of oral implantology is well known; this consists of the setting in place of dental implants, normally manufactured from titanium, to simulate the root in a place where there are teeth missing. The complexity of said technique is conditioned, for example, by factors such as the number of implants to be set, the length, diameter and quality of the bone where they will be seated, and also the characteristics of the chosen prosthesis.

Subsequent to a personalised, careful study performed by specialists, the implants are set in position by means of an intervention under local anaesthetic. Occasionally, depending on the implant system used, a second surgical intervention is necessary. On completion of a period of time subsequent to the intervention, this being necessary in order to make possible the joining between the bone and the implant (osteointegration), the prosthesis is prepared from the impressions made with accessories manufactured to this end (transfers), which are set and screwed into the heads of the implants.

In order to carry out the majority of the operations described, many types of tools are used, each of these being adapted to carry out a specific operation with the precision required for the same. For example, milling tools or surgical burs are used to carry out cutting operations, creating perforations in the jaw tissue in those areas where it is foreseen that the corresponding implants will be inserted.

Specifically, the making of a hole in the jaw tissue for the setting of an implant of a particular diameter implies the use of several normally motor-driven milling tools, starting with a small diameter bur followed by others of progressively greater diameters until the value of the approximate diameter of the implant to be set is reached.

In addition to the burs described above, other types of burs are for example the countersink bur used to widen the hole made in the jaw tissue prior to the fitting of the implant, or the finishing bur used subsequent to the setting of the implant per se.

The tools which are specific to the milling or boring operations are constituted of a series of burs or blades suitably spaced apart and which operate one after the other in the shaping or milling machine, the assembly performing a continuous circular movement.

In a similar way, during the screwing operations, in which a tightening force must be exerted on the implant, the use of tools adapted to be coupled to the head of the implant and to transmit to the same a torque which generates the necessary impulse to make it rotate and to insert it into the area of the jaw intended for its fixing is common.

An important aspect to be borne in mind is that, in order to prevent the overheating of both the milling tool and the jaw tissue, those skilled in the art recommend the use of the burs while exerting intermittent pressure on the surfaces to be shaped. Even so, it is advisable to adopt additional measures, such as, for example, the use of coolants, thus preventing both mechanical failures and necrotization processes.

Faced by the variety of the operations to be performed in an intervention of oral implantology, in order not to need a plurality of different tools, the use of a series of interchangeable heads is frequent; these are coupled to a single instrument, generally motor-driven in order to minimise effort and to reduce the time of the intervention. Thus, the interchangeable tool heads are coupled to a motor shaft by means of a specially designed coupling system.

By way of an example, the document of the American patent US2006210949 describes a drill head, adapted in order to facilitate the guiding and the drilling of the bone of a human jaw intended to receive a dental implant, this being preferably of a threaded shape. The different sections along the length of said drill head allow a smaller number of drilling tools to be used. However, said drilling head must be replaced by others for operations which are different from that of drilling, such as for example the making of holes of different magnitudes, or in order to carry out the milling operations which are necessary for the polishing of the holes.

The main drawback of the coupling systems used in tools such as those described, in particular in milling burs, lies in that each time, a fine duct from the coolant tank must be fitted to each tool head, a procedure which increases unnecessarily the duration of the intervention.

Specifically, the coupling ends of the motor shaft and the tool head are usually equipped with corresponding mutual fitting features, constituted essentially of a male and a female section respectively, which when fitted together are adjustedly coupled together, with no possibility of rotation between the two, in such a way that the motor shaft, when it is in motion, drives the tool head which moves solidarily with said motor shaft. The mutual fitting items further comprise a retaining means which hinders the separation of the motor shaft from the tool head, for example by means of the use of locking items which, in the coupled position, obstruct the separation of the two, in such a way that their accidental uncoupling is prevented.

The main disadvantage of this type of alternatives is that each time a change of tool is required, be it for maintenance or in order to use another different tool head, the locking item must first be released and/or even removed before uncoupling the tool from the intermediate rod.

For these reasons, the operation of interchanging tool heads used by the known coupling systems is usually highly complicated. Besides, the couplings described present the difficulties derived from the use of a greater number of separable parts, such as the lock, which increases both costs and the risk of a use which is not totally appropriate. In point of fact, during a normal intervention, the tool head is usually changed approximately twenty times on average, due to the many applied techniques which make use of both milling burs of different configurations and of tools for tightening screws. As a result, bearing in mind that each change of head requires an approximate time of one minute, the duration of the intervention may for this reason be increased by approximately twenty minutes, this time being wasted, as no action is being carried out on the patient.

### Explanation of the invention

With the purpose of providing a solution to the problems expounded, a removable coupling system is disclosed, for the execution of milling, threading, screwing on and/or unscrewing operations, for motor operated surgical tools, of the type which are comprised of two complementary coupling items, essentially constituted of a motor shaft and a tool head.

In essence, the removable coupling system which is the object of this invention is characterised in that said motor shaft and said tool head are joined together by means of an intermediate coupling rod, this being coupled to the motor shaft at one end and receiving the coupling of the tool head at the other, the intermediate coupling rod and the tool head being provided at their coupling ends with corresponding mutual fitting items constituted essentially of a male and female part respectively which, when fitted together are adjustedly coupled together, with no possibility of turning between them, and in that it comprises elastic means which are complementary to retaining means integrated respectively into the intermediate rod and into the tool head, or vice versa, in such a way that during the coupling operation the elastic means are inserted as a press-fit into the retaining means until they reach a final coupled position in which the retaining means present resistance to the axial separation of the two parts. In this way, the elastic means, together with the retaining means, act as an automatic locking element and maintain the tool head coupled to the intermediate rod which is now fixed to the motor shaft, with no need for the operation of an external locking element.

In accordance with another characteristic of the invention, the elastic means consist of an elastic ring washer open at one of its sections, establishing two ends and arranged on the male section; and in that the retaining means is comprised of a hollow press-fit section and a groove arranged in the female part, these parts being essentially cylindrical, the diameter of the hollow press-fit section being noticeably smaller than the external diameter of the open elastic ring washer.

In accordance with another characteristic of the invention, the open elastic ring washer is of a metallic nature, and the open elastic ring washer shall preferably be of stainless steel.

In accordance with another characteristic of the invention, the open elastic ring washer has a cross-section whose external outline has chamfered sections, intended to facilitate the coupling between the male and female parts of the intermediate coupling rod and the tool head, respectively.

In accordance with another embodiment of the removable coupling system which is the object of this invention, the elastic means is an elastic rubber item of an essentially toroidal shaped configuration, and the retaining means consists of a hollow press-fit section in the female part, this being essentially cylindrical and of a diameter noticeably smaller than the outside diameter of the toroidal shaped elastic rubber item.

In accordance with another characteristic of the invention, the elastic rubber item of the elastic means is made of surgical silicone.

Another notable aspect of the removable coupling system which is the object of the invention is that the female section links to a hole whose end is at the exterior of the tool head, this hole being the prolongation of an axial hole throughout the whole length of the intermediate rod in which a cooling duct is at least partially housed.

Thanks to these characteristics, when a change of tool is required, it is only necessary to change the tool head on the intermediate rod, which remains coupled to the motor shaft.

### Brief description of the drawings

In the attached drawings, two embodiments of the removable coupling system which is the object of the invention are portrayed, as a non-limitative example. In said drawings:
Figures 1 and 2 are respective perspective views of an assembly consisting of a tool head and intermediate coupling rod, which comprise a first embodiment of the removable coupling system of this invention, the two components being in the assembled position;
Figure 3 is a perspective view of the assembly in Figures 1 and 2, in a disassembled position;
Figure 4 is a detailed view of the elastic means of the intermediate rod in Figure 3;
Figure 5 is a sectional view of the assembly consisting of the tool head and the intermediate coupling rod in Figure 3 in accordance with a longitudinal section plane;
Figure 6 is a sectional view of a detail of the elastic means of the removable coupling system, the assembly being in the coupled position as in Figures 1 and 2, in accordance with a longitudinal section plane;
Figure 7 is a perspective view of another tool head and intermediate coupling rod assembly, which comprises a second embodiment of the removable coupling system of this invention, both parts being in the uncoupled position;
Figure 8 is another perspective view of the assembly in Figure 6, in which the tool head and the intermediate rod are coupled; and
Figure 9 is a sectional view in accordance with a longitudinal cutting plane of the assembly in Figure 1.

### Detailed description of the drawings

In surgical operations in the field of dental implantology, the handling of a great number and variety of surgical tools is required, such as for example motorised milling burs of different hole diameters, in order to make a hole in the jaw tissue which is suitable for the setting of the dental implant.

In order to save time and effort, instead of changing continually from one bur to another, of increasing diameters, it being necessary to fit to the surgical tool, at each change, the fine coolant duct whose purpose it is to cool both the bur and the area of the jaw in which the corresponding hole is being drilled, some motorised surgical tools feature complementary coupling items between a motor shaft 20 and a plurality of tool heads 2 (of different configurations and diameters),

This invention portrays a coupling system 10 by means of which the motor shaft 20 and the tool head 2 are linked together by means of an intermediate coupling rod 1. For this to be so, the intermediate coupling rod 1 and each of the tool heads 2 are equipped at their coupling ends with corresponding mutual coupling means, essentially comprised of a male section 3 and a female section 4, respectively, which when fitted together are adjustedly coupled together with no possibility of turning between them.

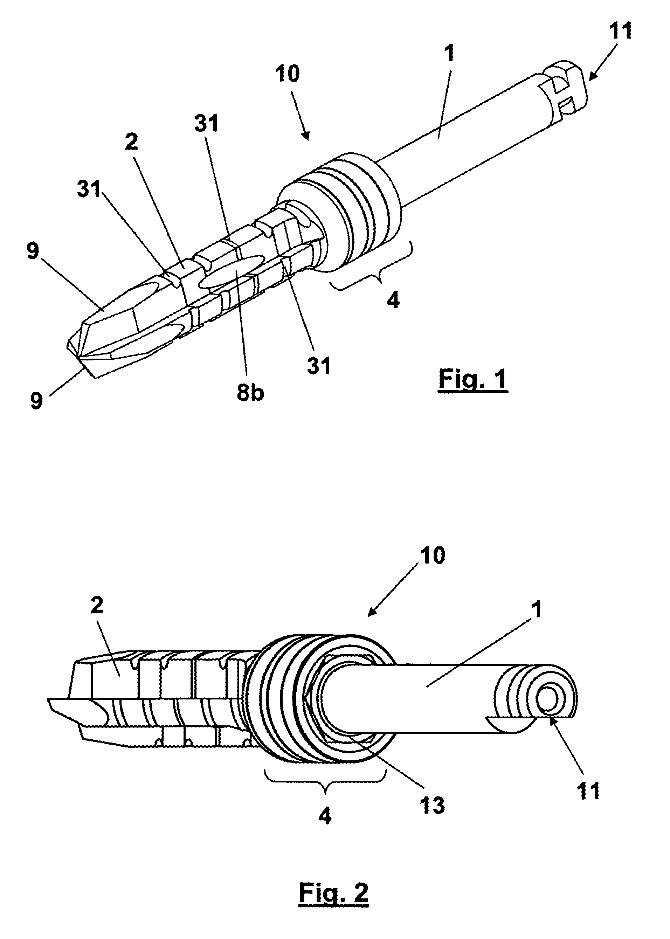

In Figures 1, 2, 3 and 5, the assembly formed by an intermediate rod 1 and a tool head 2 corresponding to the head of a surgical bur is portrayed, said assembly featuring the removable coupling system 10 in accordance with the invention. Thanks to the intermediate coupling system 10 it is possible to change the tool heads 2 on the intermediate coupling rod 1 rapidly and easily, guaranteeing a firm, safe coupling of the surgical bur while said bur is operating, as portrayed in Figures 1 and 2.

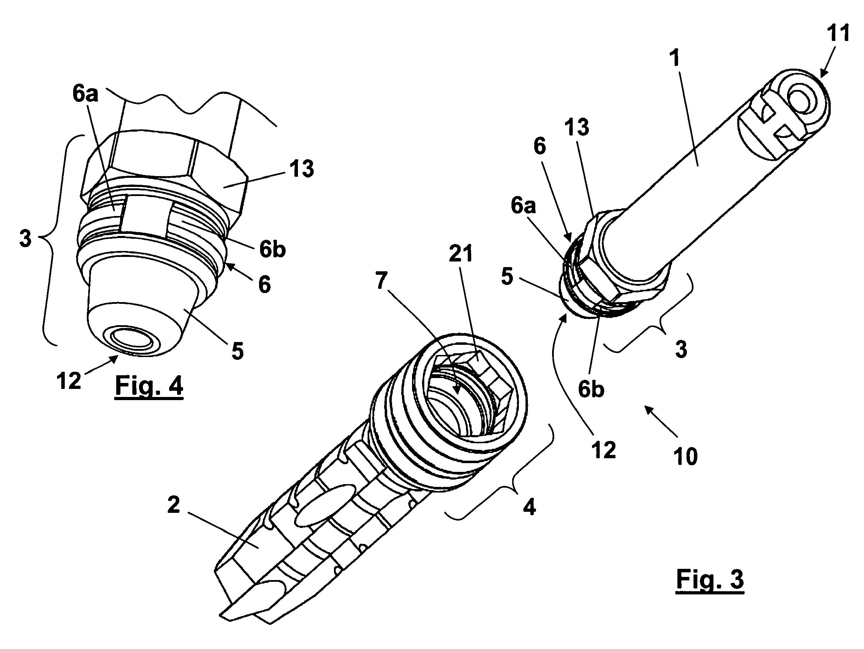

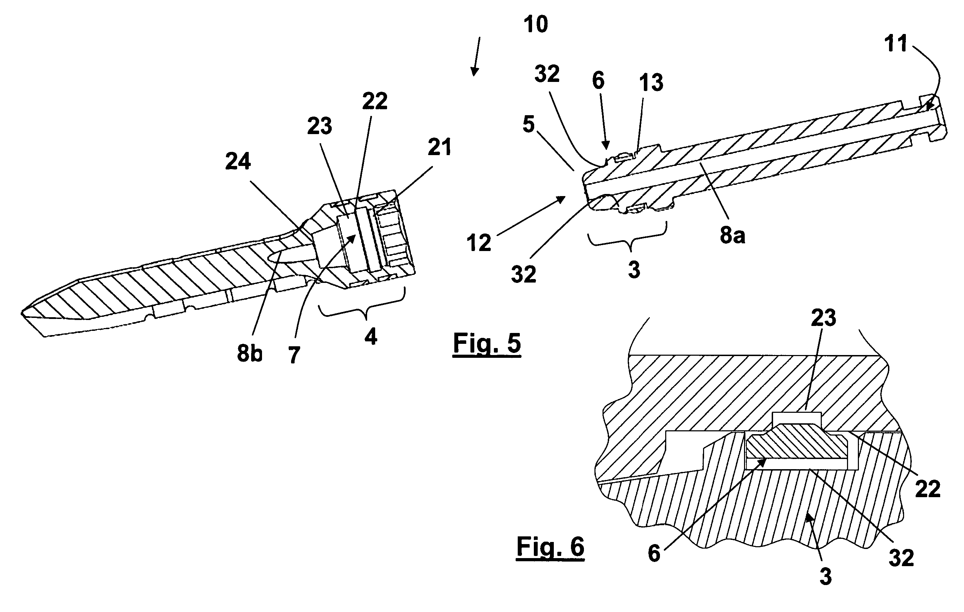

As may be seen in Figures 3 and 5, the removable coupling system 10 is comprised of an elastic means 6 which is complementary to a retaining means 7 arranged respectively on the intermediate rod 1 and in the tool head 2, in such a way that once the intermediate rod 1 and the tool head 2 are coupled together, the elastic means 6 is fitted into the retaining means 7, thus presenting resistance to the axial separation of the two parts.

It may be seen in Figures 3 to 5 that the intermediate rod 1, in addition to an end 11 which may be coupled to a motor shaft 20, features at the opposite end 12 to the motor shaft 20, the male section 3 of the coupling system 10 which comprises a locking element 13 of an essentially hexagonal shape, an elastic washer open at one of its sections, which constitutes the elastic means 6 of the removable coupling system 10, and an essentially truncated cone-shaped extension 5.

As for the tool head 2 in Figures 1, 2, 3 and 5, it constitutes a milling bur formed by four ribs 9, acting as blades and conveniently separated from each other which, on rotating around their axial axis due to the turning movement transmitted by the intermediate rod 1 driven by the motor shaft 20, drills the jaw tissue, making a hole of a radius determined by the ribs 9. In order that the surgeon may control the depth of the hole executed with the bur, the tool head 2 features five grooves 31 around its perimeter, marked in a certain colour by means of laser.

The tool head 2 features at its base a female section 4 for the coupling of the male section 3 of the rod 1. The aforementioned female section 4 features a cavity which has four different sections (see Figure 5); a first hollow fitting section 21, whose cross-section features a shape which is at least partially complementary to the locking element 13, a second essentially cylindrical hollow press-fit section 22, of a diameter noticeably smaller than that of the open elastic washer 6, a third section consisting of a groove 23, of a diameter noticeably greater than that of the open elastic washer 6, and a final truncated cone-shaped section 24 of a shape essentially complementary to that of the extension 5 of the intermediate rod 1.

In particular, the first hollow fitting section 21, the press-fit section 22 and the final section 24 of the cavity in the female section 4 of the tool head 2 are arranged in such a way that they coincide with the locking element 13, with the open elastic washer 6 and with the extension 5 respectively, when the intermediate rod 1 and the tool head 2 are mutually coupled together as portrayed in Figures 1 and 2.

Evidently, the locking element 13 may feature any polygonal outline, or may even feature an irregular curved outline with protrusions and indentations, as due to the fact that the shape of the hollow fitting section 21 is complementary, the link will be guaranteed, eliminating relative rotation between the intermediate rod 1 and the tool head 2.

Specifically, the essentially cylindrical hollow press-fit section 22 and the groove 23 constitute the retaining means 7, thanks to which the open elastic washer 6 of the intermediate rod 1 is held fast when the latter is mutually coupled to the tool head 2, as is explained below.

During the coupling operation, in which the insertion of the male section 3 of the intermediate rod 1 into the female section 4 of the tool head 2 is commenced, the open elastic washer 6 is press-fitted into the press-fit section 22 until it reaches the groove 23, its final position after coupling, in which the open elastic washer 6 is encased, occupying a part or the entirety of the space formed by the groove 23, as is portrayed in detail in Figure 6. Due to the fact that the diameter of the groove 23 is greater than that of the hollow press-fit section 22, the open elastic washer 6 is fitted and held in the groove 23, hindering the separation of the tool head 2 from the intermediate rod 1, as the open elastic washer 6, when moving in an uncoupling direction, buttresses against the wall of the groove 23 bordering the press-fit section 22. In order to separate the tool head 2 from the intermediate rod 1, the tool head 2 should be pulled in order to force the open elastic washer 6, this being lodged in the groove 23, to enter, once again under pressure, into the press-fit section 22, this time at the end opposite to that used for the coupling operation; subsequently moving through the hollow fitting section 21 until it reaches the exterior.

On the other hand, the truncated cone-shaped configuration of the extension 5 allows correct guidance when bringing together the ends of the intermediate rod 1 and the tool head 2 for their mutual coupling, until said extension 5 is finally inserted into the final section 24 of the tool head 2. Furthermore, in the coupled position portrayed in Figures 1 and 2, the extension 5 provides greater rigidity to the coupling assembly 10.

With regard to the open elastic washer 6, it may be seen in detail in Figures 3 and 4 that it is, in fact, a washer which is open at one of its sections, two ends 6a and 6b being clearly established. The aforementioned open elastic washer 6 is of a metallic nature, preferably of stainless steel, and said configuration favours its elastic properties, receiving radial pressure due to its contact with the walls of the hollow press-fit section 22 when it moves through said section of the female part 4 of the tool head 2, due to which it becomes elastically distorted until none of the sections presents resistance, and it tends to return to its initial configuration. Thus, in its uncoupled position, the ends 6a and 6b are separated from each other, while during the coupling operation, when the male section 3 of the intermediate rod 1 is inserted into the female section 4 of the tool head 2, the external configuration of the open elastic washer 6 presses against the walls of the press-fit section 22 and its ends 6a and 6b tend to come together in order to overcome the resistance and to move through said press-fit section 22, adapting itself to the diameter of the latter, this being noticeably smaller than that of the open elastic washer 6. Once the intermediate rod 1 and the tool head 2 are coupled, the open elastic washer 6, having reached the groove 23, and due to its elasticity and to its configuration, has a natural tendency to recover its initial configuration by opening, that is to say, its ends 6a and 6b tend to separate, thus being held in the groove 23 and therefore guaranteeing the coupling executed as has been described above.

It should be mentioned that, as is portrayed in Figures 5 and 6, the open elastic washer 6 is housed in a groove 32 executed in the male section 3 of the intermediate rod 1, without touching, in the absence of compression forces, the entirety of the base of said groove 32. It may also be seen that the width of the groove 32 is slightly greater than that of the open elastic washer 6, in such a way that there is a certain play in order to facilitate the coupling operation. The distance between the ends 6a and 6b, added to the diameter of the open elastic washer 6, have sufficiently small dimensions to guarantee that the open elastic washer 6 will remain always linked to the male section 3, without exiting the groove 32 on any occasion.

Although the cross-section of the open elastic washer 6 may adopt different geometric shapes, the cross-section portrayed in Figures 4, 5 and 6 with a chamfered shape, similar to a trapezium with extensions at its ends, is particularly advantageous as the slant of the chamfers facilitates a smooth coupling and uncoupling of the intermediate rod 1 with the tool head 2. In this way, the smooth guided insertion and removal of the open elastic washer 6 through the press-fit section 22 is facilitated during the coupling and uncoupling operations respectively.

The need for cooling of surgical tools has been mentioned above; this is not only due to the heating of the tool itself, but also to the transfer of heat which said tool might pass to the jaw tissue, it even being possible to cause its necrotization if it is not cooled suitably.

The removable coupling system 10 makes possible the aforementioned cooling of the surgical tool, in this case a bur, as will be explained below. In Figure 5 it may be seen that the intermediate rod 1 features an axial hole 8a throughout its entire length, intended to house in its interior a coolant supply duct. When the intermediate rod 1 is coupled to a tool head 2, the coolant which flows through the axial hole 8a continues through a second hole 8b located after the truncated cone-shaped final section 24 of the female section 4 of the tool head 2; for this purpose said final section 24 features an orifice in its end wall; through this orifice the coolant emerges to the exterior through the hole 8b. As is portrayed in Figures 1 and 3, the hole 8b has an outlet to the exterior in one of the walls which constitute the cutting lips 9 of the bur.

In this way, if during a stage of a surgical implantology intervention the surgeon needs to make three bur changes, (s)he will only need to change the tool head 2 three times, corresponding to the shapes of the burs required, coupling these heads one after the other to the same intermediate rod 1 which will already have been coupled by its end 11 to the motor shaft 20, and also to a fine coolant supply duct, since the beginning of the operation. The removable coupling system 10 allows a simple, rapid, safe coupling of the tool heads 2 to the intermediate rod 1, with no need to waste time fitting the fine coolant supply duct each time the tool or head is changed, as said duct only requires fitting once to the intermediate rod 1, to which the necessary tool heads 2 will be coupled during the course of the intervention.

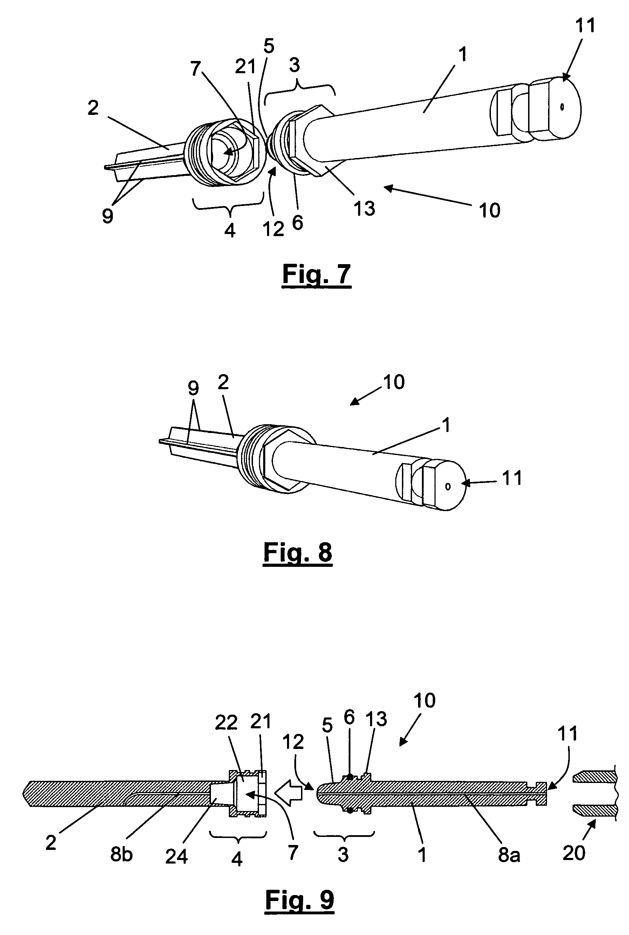

A second embodiment of the removable coupling system 10 may be seen in Figures 7 and 8, applied to a different model of bur from that mentioned above, in which the elastic means 6 are constituted of an elastic toroidal shaped rubber, manufactured from surgical silicone, a hypoallergenic, hygienic material which is sufficiently elastic so that once the tool head 2 is coupled to the intermediate rod 1, the aforementioned elastic retaining rubber is radially compressed by the retaining means 7, presenting resistance to the axial separation of the two sections due to the friction caused by the contact between the elastic retaining rubber 6 and the corresponding cylindrical hollow press-fit section 22 of the female section 4 of the tool head 2.

## Claims

**1.** A removable coupling system (10) for carrying out milling, threading, screwing on and/or unscrewing operations, for motor operated surgical tools, of the type that comprises two complementary coupling items, essentially constituted of a motor shaft (20) and a tool head (2), **characterised in that** said motor shaft and said tool head are linked together by an intermediate coupling rod (1), this being coupled at one end to the motor shaft and receiving the coupling of the tool head at the other, the intermediate coupling rod and the tool head being provided, at their coupling ends, with corresponding mutual fitting items, constituted essentially of a male (3) and a female (4) section respectively, which when fitted together are adjustedly coupled together, with no possibility of rotation between the two, and **in that** it comprises an elastic means (6) complementary to a retaining means (7) integrated respectively in the intermediate rod and in the tool head, or vice versa, in such a way that during the coupling operation the elastic means is press-fitted into the retaining means until it reaches a final coupled position in which the retaining means presents resistance to the axial separation of the two sections.

**2.** A removable coupling system (10) according to claim 1, **characterised in that** the elastic means (6) consists of an elastic washer, open at one of its sections, establishing two ends (6a, 6b), arranged on the male section (3) and **in that** the retaining means (7) comprises a hollow press-fit section (22) and a groove (23) arranged in the female section (4), these being essentially cylindrical, the diameter of the hollow press-fit section being noticeably smaller than the outside diameter of the open elastic washer (6).

**3.** A removable coupling system (10) according to claim 2, **characterised in that** the open elastic washer (6) is metallic.

**4.** A removable coupling system (10) according to claim 3, **characterised in that** the open elastic washer (6) is made of stainless steel.

**5.** A removable coupling system (10) according to any one of claims 2 to 4, **characterised in that** the open elastic washer (6) has a cross-section whose external outline comprises chamfered areas, intended to favour the coupling between the male (3) and female (4) sections of the intermediate coupling rod (1) and of the tool head (2), respectively.

**6.** A removable coupling system (10) according to claim 1, **characterised in that** the elastic means (6) consists of an elastic essentially toroidal shaped rubber and **in that** the retaining means (7) consists of a hollow press-fit section (22) in the female part (4), this being essentially cylindrical and having a diameter noticeably smaller than the outside diameter of the toroidal shaped elastic rubber.

**7.** A removable coupling system (10) according to claim 6, **characterised in that** the elastic rubber of the elastic means (6) is made of surgical silicone.

**8.** A removable coupling system (10) according to any one of claims 2 to 7, **characterised in that** the female section (4) communicates with a hole (8b) whose end is on the exterior of the tool head (2), this hole being the prolongation of an axial hole (8a) throughout the whole length of the intermediate rod (1) in which a coolant duct is at least partially housed.
